# EUROPEAN PATENT APPLICATION

(11) **EP 3 222 721 A1**
(43) Date of publication of application: **27.09.2017**
(21) Application number: 15861258.0
(22) Date of filing: 17.11.2015
(51) Int. Cl.: C12N 15/11, C12N 15/63, A61K 48/00, A61P 35/00

(54) **NOVEL PRECURSOR MIRNA AND APPLICATION THEREOF IN TUMOR TREATMENT**

(30) Priority: 17.11.2014 CN 201410652458; 05.05.2015 CN 201510224944
(71) Applicant: Jiangsu Micromedmark Biotech Co., Ltd., Taizhou, Jiangsu 225300 (CN)
(72) Inventor: ZHANG, Chenyu, Taizhou Jiangsu 225300 (CN); ZENG, Ke, Taizhou Jiangsu 225300 (CN); CHEN, Xi, Taizhou Jiangsu 225300 (CN); ZHANG, Junfeng, Taizhou Jiangsu 225300 (CN); LIANG, Hongwei, Taizhou Jiangsu 225300 (CN)
(74) Representative: Lavoix
(86) International application number: PCT/CN2015/094809
(87) International publication number: WO 2016/078572

(57) **Abstract**

Provided are a precursor miRNA and application thereof in a tumor treatment. The precursor miRNA from the 5' end to 3' end has a structure presented as formula I: 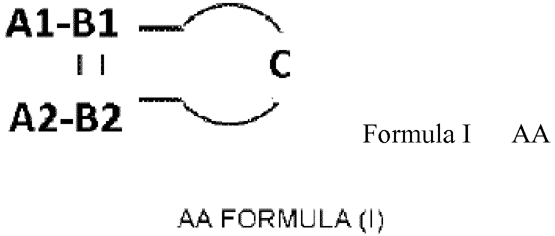 B1 is anti-miRNA-214-5p; B2 is an essentially complementary sequence or a totally complementary sequence to B1, and B2 and C are not complementary; C is a sequence having a stem-loop structure; A1 and A2 are respectively RNA sequences having no or 4-5 bases freely selected bases respectively; the precursor miRNA shown can be processed to form anti-miRNA-214 in a host, and only anti-miRNA-214-5p but not anti-miRNA-214-3p is expressed in the anti-miRNA-214.

SEQUENCE LISTING

<110>  JIANGSU MICROMEDMARK BIOTECH CO., LTD.

<120>  Novel precursor miRNAs and application thereof in tumor treatment

<130>  BET 17M1842

<140>  PCT/CN2015/094809
<141>  2015-11-17

<150>  CN201410652458.5
<151>  2014-11-17

<150>  CN201510224944.1
<151>  2015-05-05

<160>  9

<170>  PatentIn version 3.5

<210>  1
<211>  19
<212>  RNA
<213>  oligonucleotides

<400>  1
guuuuggcca cugacugac                                                    19


<210>  2
<211>  22
<212>  RNA
<213>  oligonucleotides

<400>  2
acugccuguc ugugccugcu gu                                                22


<210>  3
<211>  22
<212>  RNA
<213>  oligonucleotides

<400>  3
agagcaggca cagacaggca gu                                                22


<210>  4
<211>  61
<212>  DNA
<213>  oligonucleotides

<400>  4
tgctgactgc ctgtctgtgc ctgctgtgtt ttggccactg accaaagtat catctttgta      60

g                                                                       61


<210>  5
<211>  61
<212>  DNA
<213>  oligonucleotides

<400>  5
cctgctacaa agatgatact ttggtcagtg gccaaaacag agcaggcaca gacaggcagt        60

c                                                                        61


<210>  6
<211>  64
<212>  DNA
<213>  oligonucleotides

<400>  6
tgctgaaatg tactgcgcgt ggagacgttt tggccactga ctgacgtctc cacgcagtac        60

attt                                                                     64


<210>  7
<211>  64
<212>  DNA
<213>  oligonucleotides

<400>  7
cctgaaatgt actgcgtgga gacgtcagtc agtggccaaa acgtctccac gcgcagtaca        60

tttc                                                                     64


<210>  8
<211>  24
<212>  DNA
<213>  oligonucleotides

<400>  8
tgctggtttt ggccactgac tgac                                               24


<210>  9
<211>  24
<212>  DNA
<213>  oligonucleotides

<400>  9
cctggtcagt cagtggccaa aacc                                               24

## Description

### Technical Field

The present invention relates to the tumor treatment field, and particularly to new precursor miRNAs and a method and use thereof for inhibiting tumor growth.

### Background Art

MicroRNAs are derived from long chain RNA primary transcripts (pri-miRNAs) with a length of about 1000 bp which are cleaved in the cell nucleus by enzyme Drosha into miRNA precursors having a stem-loop structure with a length of about 60-80 nt. The precursor miRNAs are further cleaved into double-strand miRNAs with a length of about 18-26 nt after being transported to the cytoplasm. After the double-strand miRNAs are unwound, the mature miRNAs enter into RNA-induced silencing complexes (RISC), which are completely or not completely paired with the complementary mRNAs, so that the target mRNAs are degraded or the expression thereof is repressed.

MicroRNAs account for a small proportion of the total cellular RNA; however, miRNAs participate in a series of important processes in the life process, including early embryonic development, cell proliferation and cell death, apoptosis and fat metabolism, cell differentiation. Considering their role in gene expression regulation, and that abnormal cell proliferation, apoptosis and the like often occur in tumors, it is therefore presumed that abnormal deletion, mutation or over-expression of miRNAs will lead to the occurrence of human diseases.

Recently, increasing amounts of evidence shows that miRNAs play a very important role in inhibiting tumor cell growth, proliferation and differentiation. In the art, there is an urgent need to understand the roles of various different miRNAs to develop new medicaments for treating tumors.

### Summary of the Invention

The present invention provides new precursor miRNAs expressing anti-miRNA-214, and the use thereof in treating tumors.

The first aspect of the present invention provides a pharmaceutical preparation comprising:
(a) an expression vector for expressing anti-miRNAs and/or siRNAs; and
(b) a pharmaceutically acceptable carrier.

In another preferred example, with regard to the precursor as shown in formula I-1 expressed by the expression vector, 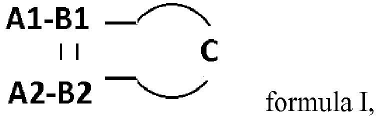
B1 is a first ribonucleic acid sequence as desired, and comprises an anti-miRNA sequence form or a siRNA sequence form;
B2 is a sequence substantially or completely complementary to B1, and B2 is not complementary to C;
C is a stem-loop structure sequence; and
A1 and A2 are null or are optionally RNA sequences consisting of 4-5 bases respectively;
Wherein, the said precursor can express (or produce or enrich) the said first ribonucleic acid sequence in a host, but cannot express (or produce or enrich) a second ribonucleic acid sequence complementary to the said first ribonucleic acid sequence.

In another preferred example, the said first ribonucleic acid sequence is in the form of 5p, and the second ribonucleic acid sequence in the form of 3p.

In another preferred example, the said first ribonucleic acid sequence is in the form of 3p, and the second ribonucleic acid sequence in the form of 5p.

In another preferred example, the said first ribonucleic acid sequence is anti-miRNAs and/or siRNAs.

In another preferred example, the said B1 is miRNAs or siRNAs against tumors.

In another preferred example, the said B1 is anti-miRNA-214-5p.

In another preferred example, the first ribonucleic acid sequence is anti-miRNA-214-5p, and the second ribonucleic acid sequence is anti-miRNA-214-3p.

In another preferred example, the said preparation is in a liquid dosage form.

In another preferred example, the said preparation is an injection.

In another preferred example, the said expression vector comprises a plasmid.

In another preferred example, the said expression vector or plasmid contains a promoter, a replication origin and a marker gene.

In another preferred example, the said expression vector contains an expression cassette expressing anti-miRNAs and/or siRNAs.

In another preferred example, the said expression cassette (i.e. a polynucleotide) is double-stranded and has the following structure:
a promoter-attB1-an optional tag protein (such as GFP or emGFP)-a 5'miR flanking region sequence-the sequence as shown in formula I-a 5'miR flanking region sequence-attB2-an optional TKPA element.

In another preferred example, the said preparation is a liposome preparation.

The second aspect of the present invention provides a method for administering a medicament, comprising the step of:
To administer the said pharmaceutical preparation of the first aspect of the present invention at a first body site of a mammal, so that the expression vector is processed *in vivo* to form microvesicles, which are transported to a second body site of the mammal where the anti-miRNAs and/or siRNAs are expressed.

In another preferred example, the said mammal includes human and non-human mammals.

In another preferred example, the said first site includes a subcutaneous, intravenous or gastrointestinal tract site.

In another preferred example, the said second site includes the liver, lung, and kidney.

In another preferred example, the said administering includes oral intake, subcutaneous injection, intramuscular injection and intravenous injection.

The third aspect of the present invention provides a precursor sequence having a structure from the 5' terminus to the 3' terminus as shown in formula I: 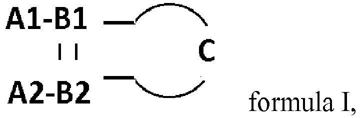
Wherein, B 1 is a first ribonucleic acid sequence as desired and comprises an anti-miRNA sequence form or a siRNA sequence form;
   B2 is a sequence substantially or completely complementary to B1, and B2 is not complementary to C;
   C is a stem-loop structure sequence; and
   A1 and A2 are null, or are optionally RNA sequences consisting of 4-5 bases respectively;
   Wherein, the said precursor can express (or produce or enrich) the said first ribonucleic acid sequence in a host, but cannot express (or produce or enrich) a second ribonucleic acid sequence that is complementary to the said first ribonucleic acid sequence.

The fourth aspect of the present invention provides a precursor sequence having a structure from the 5' terminus to the 3' terminus as shown in formula I: 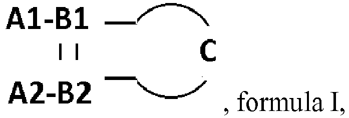
Wherein, B1 is anti-miRNA-214-5p;
B2 is a sequence substantially or completely complementary to B1, and B2 is not complementary to C;
C is a stem-loop structure sequence, preferably a sequence shown as SEQ ID NO.: 1 (GUUUUGGCCACUGACUGAC); and
A1 and A2 are null, or are optionally RNA sequences consisting of 4-5 bases respectively;
Wherein, the precursor sequence as shown can be processed in the host into anti-miRNA-214, and only anti-miRNA-214-5p, rather than anti-miRNA-214-3p, in the said anti-miRNA-214 is expressed.

In another preferred example, the wording "only anti-miRNA-214-5p, rather than anti-miRNA-214-3p, is expressed" means that the ratio of F5/F3 is ≥ 5, preferably ≥ 10, more preferably ≥ 20, and most preferably ≥ 50, wherein F5 is the expression amount of anti-miRNA-214-5p and F3 is the expression amount of anti-miRNA-214-3p. In another preferred example, the said anti-miRNA-214-5p is shown as SEQ ID NO.: 2 (ACUGCCUGUCUGUGCCUGCCUGU).

In another preferred example, the said anti-miRNA-214-3p is shown as SEQ ID NO.: 3 (ACAGGCAGGC AGACAGGCAGU).

In another preferred example, there are 2-8 non-complementary bases between the said B2 and B1, preferably, 3-5 non-complementary bases between the said B2 and B1.

In another preferred example, 1-2 bases are added or deleted in the said B2 as compared with B 1.

In another preferred example, 1-2 bases, preferably 2 bases, are deleted in the said B2 as compared with B 1.

In another preferred example, the deleted 1-2 bases are in the middle of B1, i.e., 1-2 bases at positions 9-14, such as positions 9-10, 10-11, 11-12, 12-13 or 13-14.

In another preferred example, the said A1 is UGCUG; and/or
the said A2 is CAGG or CAGGA.

In another preferred example, A2 is preferably CAGG.

The fifth aspect of the present invention provides a polynucleotide, which can be transcribed by a host to form the precursor sequence as said in the fourth aspect of the present invention.

In another preferred example, the said polynucleotide is double-stranded and has the following structure:
attB1-a optional tag protein (such as GFP or emGFP)-a 5'miR flanking sequence-the sequence as shown in formula I-a 5'miR flanking sequence-attB2.

The sixth of the present invention provides an expression vector containing the precursor sequence as said in the fourth aspect of the present invention or the polynucleotide as said in the fifth aspect of the present invention.

In another preferred example, the said expression vector includes viral vector and non-viral vector.

In another preferred example, the said expression vector is a plasmid.

In another preferred example, the upstream of the polynucleotide as said in the fifth aspect of the present invention is a promoter, and the downstream thereof is a TKPA element.

The seventh aspect of the present invention provides a pharmaceutical composition comprising the precursor sequence as said in the fourth aspect of the present invention or the expression vector as said in the sixth aspect of the present invention, and a pharmaceutically acceptable carrier.

In another preferred example, the said pharmaceutical composition comprises the anti-miR-214 plasmid.

In another preferred example, the said pharmaceutical composition is the expression vector of the sixth aspect as said in the present invention, and preferably, a plasmid containing the precursor sequence as said in the fourth aspect of the present invention.

In another preferred example, the dosage form of the said pharmaceutical composition includes:
a tablet, a capsule, a powder, a pill, a granule, a syrup, a solution, a suspension liquid, an emulsion, a suspension, an injection solution, or an injectable powder.

In another preferred example, the dosage form of the said pharmaceutical composition also comprises a spray, an aerosol, a powder spray, a volatile liquid, a topical solution, a lotion, a pour-on agent, a liniment, a cataplasma, a medicinal paste, a rubber paste, an ointment, a plaster, a paste, an eye drop, a nasal drop, an ophthalmic ointment, a mouth wash, a sublingual tablet, or a suppository.

In another preferred example, the said dosage form is an injection, preferably an intravenous injection or an intraperitoneal injection.

The eighth aspect of the present invention provides the use of the precursor sequence as said in the fourth aspect of the present invention or the expression vector as said in the sixth aspect of the present invention, including (i) for preparing an inhibitor of miRNA-214; and/or (ii) for preparing a pharmaceutical composition against malignant tumors with high expression of miRNA-214.

In another preferred example, the said malignant tumors include liver cancer, lung cancer, stomach cancer, oesophageal cancer, ovarian cancer, colorectal cancer, cervical cancer, pancreatic cancer, prostatic cancer, leukaemia or breast cancer.

The ninth aspect of the present invention provides a method for inhibiting the growth of malignant tumor cells with high expression of miRNA-214 in a non-therapeutic manner *in vitro*, comprising the step of:
Culturing the malignant tumor cells with high expression of miRNA-214 in the presence of the pharmaceutical composition as said in the seventh aspect of the present invention, so as to inhibit the growth of malignant tumor cells with high expression of miRNA-214.

The tenth aspect of the present invention provides a method for treating malignant tumors with high expression of miRNA-214, which involves administering a safe and effective amount of the expression vector as said in the sixth aspect of the present invention or the pharmaceutical composition as said in the seventh aspect of the present invention to a subject in need, so as to treat malignant tumors with high expression of miRNA-214.

In another preferred example, the dosage of the said administering is 0.05-10 mg/kg, preferably 0.1-5 mg/kg.

In another preferred example, the manners of the said administering include oral, respiratory tract, injection, transdermal, mucosal, or cavity administration.

In another preferred example, the said administering comprises a plasmid injection.

The eleventh aspect of the present invention provides a method for treating malignant tumors with high expression of miRNA-214, characterised in that the method involves administering the anti-miR-214 plasmid containing the precursor sequence as said in the fourth aspect of the present invention by intravenous injection to a subject in need, so as to treat malignant tumors with high expression of miRNA-214.

It should be understood that all of the various technical features described above and specifically described hereinafter (such as in the examples) can be combined with one another within the scope of the present invention, so as to form new or preferred technical solutions. Due to space limitations, these are no longer tired out one by one.

### Description of the Drawings

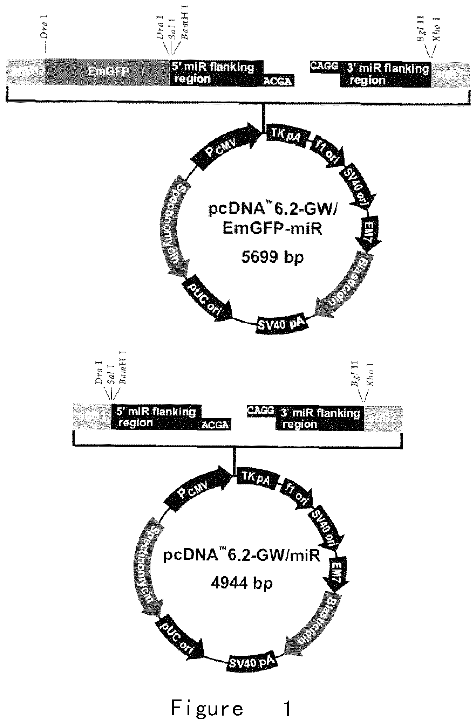

Figure 1 shows a representative plasmid profile of the present invention, wherein the "flanking region" represents a flanking region (sequence).
Figure 2 shows the siRNA amounts in various tissues or visceral organs of mice detected at time points of 1 h, 3 h, 6 h, 24 h and the like after the anti-miR-214 overexpressing-plasmids are administered by intravenous tail injection.
Figure 3 shows the CD4⁺ T cell count changes.
Figure 4 shows the amounts of anti-miR-214 in various tissues.
Figure 5 shows the effect of anti-miR-214 on the weight of tumor-bearing mice.
Figure 6 shows tissue section images illustrating the therapeutic effect of anti-miR-214 on Lewis lung cancer mice, wherein A: ×40; B: ×100; C: ×400.
Figure 7 shows the amounts of anti-miR-214 in blood cells and plasma of volunteers with end-stage tumors before and after plasmid injection.
Figure 8 shows the expression level of miR-214 in serum.
Figure 9 shows the pcDNA3 plasmid profile structure.

### Particular Embodiments

The inventors firstly designed and prepared miRNA precursors capable of efficiently expressing anti-miRNA-214 by extensive and deep studies. The precursor miRNAs of the present invention, after having been processed by a host cell, can efficiently express anti-miRNA-214-5p, without producing or substantially producing anti-miRNA-214-3p, so as to effectively avoid the interference effect of the reverse complementary sequence of a target sequence on the functioning of the target sequence. The experiment demonstrated that the precursor miRNAs of the present invention can efficiently express the anti-miRNA-214-5p sequence, and has a more effective therapeutic effect on various malignant tumors. The present invention is accomplished on this basis.

### MiRNAs and their precursors

As used herein, the "miRNAs" refer to a class of RNA molecules, which are originated from miRNA precursors that are products of transcript procession. The mature miRNAs generally have 18-26 nucleotides (nt) (more specifically, about 19-22nt), not excluding miRNA molecules having other numbers of nucleotides. MiRNA can generally be detected by Northern blotting.

The miRNAs derived from humans can be isolated from human cells. As used herein, "isolated" means that the substance is isolated from its original environment (if it is a natural substance, the original environment is the natural environment). For example, polynucleotides and polypeptides in the natural environment of living cells are not isolated and purified, but when the same polynucleotides or polypeptides are isolated from other substances coexisting in the natural environment, they are isolated and purified.

MiRNAs can be obtained by processing the precursor miRNAs (pre-miRNAs), and the precursor miRNAs can be folded into a stable stem-loop (hairpin) structure having a general length of 50-100bp. The precursor miRNAs can be folded into a stable stem-loop structure, and two sides of the stem of the stem-loop structure contain two sequences substantially complementary to each other.

In the present invention, the precursor miRNAs are artificially synthesised precursor miRNAs, and the precursor miRNAs have the structure as shown in formula I: 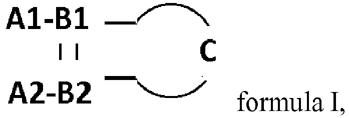
Wherein, as a representative example, B1 is anti-miRNA-214-5p;
B2 is a sequence complementary (comprising substantially and completely complementary) to B1;
C is a sequence shown as SEQ ID NO.: 1 (GUUUUGGCCACUGACUGAC);
A1 and A2 are null or are optionally nucleotide sequences consisting of 4-5 bases respectively;
Wherein, the precursor miRNAs as shown can be processed in the host to form anti-miRNA-214, and only the anti-miRNA-214-5p, rather than the anti-miRNA-214-3p, of the anti-miRNA-214 is expressed.

In the present invention, the precursor miRNAs forming the anti-miRNA-214-5p can be cleaved to generate miRNAs antagonistic to miRNA-214, i.e. anti-miRNA-214-5p (SEQ ID NO.: 2).

In formula I, B2 and B1 are substantially complementary to each other. As used herein, "substantially complementary" means that the nucleotide sequence is sufficiently complementary and that same can act upon each other in a predictable manner, e.g., forming a secondary structure (such as a stem-loop structure). Generally, at least 70% of nucleotides in two "substantially complementary" nucleotide sequences are complementary; preferably, at least 80% of nucleotides are complementary; and more preferably, at least 90% of nucleotides are complementary. Generally, there are at most 8 non-matched nucleotides, preferably 1, 2, 3, 4 and 5 non-matched nucleotides, between two sufficiently complementary molecules.

As used in the present application, the "stem-loop" structure, also known as the "hairpin" structure, refers to a nucleotide molecule which can form a secondary structure comprising a double-stranded region (stem) formed of two regions (on a same molecule) of this nucleotide molecule, the two regions being at two sides of the double-stranded portion; and the structure further comprises at least one "loop" structure, including non-complementary nucleotide molecules, i.e., a single-stranded region. Even if the two regions of the nucleotide molecule are not completely complementary, the double-stranded part of the nucleotide can also maintain the double-stranded form. For example, insertion, deletion, substitution or the like may lead to a non-complementary small region or make the small region itself form a stem-loop structure or another form of secondary structure. However, the two regions can still be substantially complementary to each other and act upon each other in a predictable manner to form a double-stranded region of the stem-loop structure. The stem-loop structure is well known to a person skilled in the art, who can generally determine, when given a nucleic acid having a nucleotide sequence of the primary structure, whether the nucleic acid can form a stem-loop structure.

In the present invention, a "stem-loop structure" can be present at the end of the precursor miRNAs as shown in formula I, for example, after B1 and B2 form a substantially complementary structure, C will form a stable stem-loop structure at the end thereof; the "stem-loop structure" can also be present in the interior of the precursor miRNAs as shown in formula I, for example, since B1 and B2 are not completely complementary, the bases of B 1 or B2 which do not bind with the others in a complementary manner will form an internal loop.

The miRNA-214 of the present invention refers to the microRNA-214 (miRNA-214) family comprising miRNA-214 or modified miRNA-214 derivatives which function identically or substantially identically to miRNA-214.

Referring to the miRNA sequences provided in the present invention, polynucleotide constructs, which can, after introduction, be processed into miRNAs capable of affecting the expression of the corresponding mRNAs, can be designed, i.e., the polynucleotide constructs can up-regulate the level of the corresponding anti-miRNA-214-5p *in vivo* so as to decrease the level of miRNA-214. Therefore, the present invention provides an isolated polynucleotide (construct), and the polynucleotide (construct) can be transcribed by human cells into precursor miRNAs which can be cleaved and expressed as the miRNAs in human cells.

### Polynucleotide constructs

As a preferred mode of the present invention, the polynucleotide construct contains a structure from the 5' terminus to the 3' terminus as shown in formula II:

a1-b1-c-b2-a2 formula II

in formula II,
b1 is a nucleotide sequence which can be expressed as the anti-miRNA-214-5p in a cell, b2 is a nucleotide sequence substantially or completely complementary to b1; c is a spacer sequence between b1 and b2, and the spacer sequence is not complementary to B1 and B2;
a1 and a2 are null or are optionally nucleotide sequences consisting of 4-5 bases respectively;
and after being introduced into the cell, the structure as shown in formula II forms a secondary structure as shown in formula I:

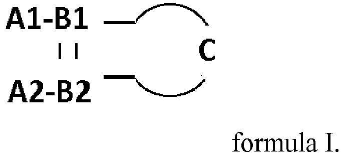

Generally, the polynucleotide constructs are located on the expression vector. Therefore, the present invention further comprises a vector containing the miRNAs or the polynucleotide constructs. The expression vector typically further contains a promoter, an origin of replication and/or a marker gene, etc. Methods well known to a person skilled in the art can be used to construct the expression vector required by the present invention. These methods include *in vitro* recombinant DNA technology, DNA synthesis technology, *in vivo* recombination technology, etc. The expression vector preferably contains one or more selectable marker genes to provide a phenotypic trait for the selection of transformed host cells, such as kanamycin, gentamicin, hygromycin or ampicillin resistance.

In the present invention, there is no special limitation on the expression vector, comprising commercially available or conventionally prepared expression vectors. Representative examples include (but are not limited to): pcDNATM6.2-GW/miR, pcDNA3, pMIR-REPORT miRNA, pAdTrack-CMV, pCAMBIA3101+pUC-35S, pCMVp-NEO-BAN, pBI121, pBin438, pCAMBIA1301, pSV2, a CMV4 expression vector, pmiR-RB-Report™, pshOK-basic, mmu-mir 300-399 miRNASelect™, pshRNA-copGFP Lentivector, GV317, GV309, GV253, GV250, GV249, GV234, GV233, GV232, GV201, GV159 or other expression vectors of the GV series.

In another preferred example, in the said expression vector, the promoter operably linked to the polynucleotide expressing the precursor miRNAs comprises a constitutive promoter or a tissue-specific promoter, preferably a promoter specifically performing initiation in liver tissues. In other words, these promoters are used to drive the expression of the precursor miRNAs.

Representative promoters include (but are not limited to): a Pcmv promoter, U6, H1, a CD43 promoter, a CD45 (LCA) promoter, a CD68 promoter, an Endoglin (CD105) promoter, a Fibronectin promoter, a Flt-1 (VEGFR-1) promoter, a GFAP promoter, a GPIIb (Integrin αIIb) promoter, an ICAM-2 (CD102) promoter, a MB (Myoglobin) promoter, a NphsI (Nephrin) promoter, a SPB promoter, a SV40/hAlb promoter, a SYN1 promoter, a WASP promoter or a combination thereof.

### Pharmaceutical composition and administration methods

As used herein, the term "effective amount" or "effective dose" refers to the amount which can induce a function or activity in humans and/or animals and can also be acceptable to humans and/or animals.

As used herein, the term "pharmaceutically acceptable" component is applicable to humans and/or mammals without excessive adverse side effects (such as toxicity, irritation and allergic responses), i.e., a substance with a reasonable benefit/risk ratio. The term "pharmaceutically acceptable carrier" refers to a carrier for the administration of a therapeutic agent, including various excipients and diluents.

The pharmaceutical composition of the present invention contains a safe and effective amount of the active component of the present invention and a pharmaceutically acceptable carrier. Such carrier includes, but is not limited to, saline, a buffer, glucose, water, glycerol, ethanol, and a combination thereof. Generally, a pharmaceutical preparation shall match the administration mode, and the dosage form of the pharmaceutical composition of the present invention can be an injection, an oral preparation (a tablet, a capsule, or an oral liquid), a transdermal agent, or a slow release agent. For example, preparation thereof is performed by a conventional method using physiological saline or an aqueous solution containing glucose and other adjuvants. The pharmaceutical composition is preferably produced under sterile conditions.

The effective amount of the active component of the present invention may vary depending on the administration mode and the severity of the disease to be treated. A person skilled in the art could determine the selection of the preferred effective amount depending on various factors (e.g., by clinical trials). The factors include, but are not limited to, the pharmacokinetic parameters of said active component, e.g., the bioavailability, metabolism, half-life, etc.; and the severity of the patient's disease to be treated, the patient's weight, the patient's immune state, the administration route, etc. Generally, when the active component of the present invention is administered at a dose of about 0.00001-50 mg/kg body weight (preferably 0.0001-10 mg/kg body weight) per day, satisfactory results can be achieved. For example, due to the urgent requirements of the treatment status, several separate doses can be administered daily, or the dosage is reduced proportionally.

The pharmaceutically acceptable carrier of the present invention includes (but is not limited to): water, saline, liposomes, lipids, micro particles, micro vesicles, exosomes, shedding vesicles, nanocapsules/nanoparticles, β-cyclodextrin capsule β-cyclodextriniclusion compound) proteins, protein-antibody conjugates, peptides, cellulose, nanogels, or a combination thereof. The choice of carriers should match the administration mode, which would be well known to a person skilled in the art.

In the present invention, the expression vector can be directly administered to a subject, and the expression vector can also be administered by preparing same into a pharmaceutical composition with a pharmaceutically acceptable carrier. The administration comprises an intravenous injection.

### Therapeutic method

The present invention further provides a method for treating malignant tumors highly expressing miRNA-214, i.e., administering a safe and effective amount of the expression vector or the pharmaceutical composition of the present invention to a subject in need, so as to treat malignant tumors highly expressing miRNA-214. Generally, "malignant tumors highly expressing miRNA-214" means that the expression amount E1 of miRNA-214 in the tumors is significantly different from the amount E0 of miRNA-214 in para-carcinoma tissues or normal tissues, and preferably, the wording "highly expressing" means that E1 ≥ 1.5 E0, more preferably E1 ≥ 2 E0. In tumor tissues, whether miRNA-214 is highly expressed can be detected through conventional methods. Generally, the malignant tumors highly expressing miRNA-214 include (but are not limited to) liver cancer, lung cancer, stomach cancer, oesophageal cancer, ovarian cancer, colorectal cancer, cervical cancer, pancreatic cancer, prostatic cancer, leukaemia or breast cancer.

### Beneficial effects of the present invention

The precursor miRNAs of the present invention can effectively avoid the over-expression of a target sequence and also the over-expression of the reverse complementary sequence of the target sequence at the same time, so as to effectively avoid the interference effect of the reverse complementary sequence of a target sequence on the functioning of the target sequence.

The precursor miRNAs of the present invention can effectively express the anti-miRNA-214-5p sequence *in vivo,* and have an effective therapeutic effect on various malignant tumors, so that the precursor miRNAs can be used for developing new medicaments for treating tumors.

### Example 1: The construction of anti-miR-214 over-expression vector

MicroRNA vectors can rapidly, efficiently and sustainably express pre-miRNAs using the CMV promoter in the cell of various mammals, the pre-miRNAs form small hairpin pre-miRNAs (about 70 nt) by the action of Drosha (RNaseIII), which form mature microRNAs (about 22 nt) by the action of Dicer, and the mature microRNAs act on target mRNAs and function to regulate the expression. By using optimised vectors, the expression module of anti-miR-214 is simulated, thereby effectively avoiding the problem that -3p and -5p are produced when some endogenous miRNAs are expressed.

### 1. Anti-miR-214-5p sequence

> anti-miR-214-5p
   5'-ACUGCCUGUCUGUGCCUGCCUGU-3' (SEQ ID NO.: 2)

### 2. Anti-miR-214 vector

### 2.1. Design and synthesis of Oligo DNAs

According to the gene sequences, 2 pairs of complementary oligo DNAs were designed and synthesised (for the oligo design method and framework, reference can be made to point 6 of the product instructions), and for the sequences, reference can be made to Table 1.

The designed and synthesised oligo structures are as follows:

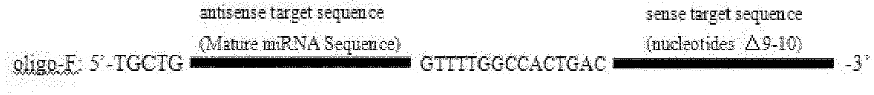
antisense target sequence
(Mature miRNA Sequence)
oligo-F: 5'-TGCTG GTTTTGGCCACTGAC -3'
sense target sequence
(nucleotides △9-10)

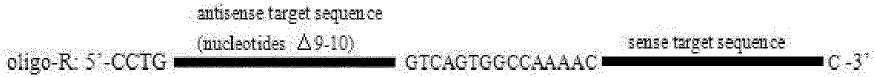
antisense target sequence
(nucleotides Δ9-10)
oligo-R: 5'-CCTG GTCAGTGGCCAAAAC C -3'
sense target sequence

**Table 1. The oligo DNA sequences and their corresponding precursor miRNA elements**

| Oligo name | Oligo DNA sequence 5'-3' |
|---|---|
| Mature sequence of >anti-miR-214-5p: 5'-ACUGCCUGUCUGUGCCUGCCUGU-3' (SEQ ID NO.: 2) | |
| 13MR0041-1F | TGCTG ACTGCCTG TC TG TGCC TGCCTG TGTTTTGGCCACTGACc aaagt atc atc tttt gt ag ; A1 ; B1 ; C ; B2 |
| | (SEQ ID NO.:4, TGCTGACTGCCTGTCTGTGCCTGCCTGTGTTTTGGCCACTGACcaaagtatcatctttgtag) |
| 13MR0041-1R | CCTGctacaaagatgatactttgGTCAGTGGCCAAAACACAGGCAGGCAGACAGGCAGTC ; A2 ;          B2          ;          C          ;          B1          ; |
| | (SEQ ID NO.: 5, CCTGctacaaagatgatactttgGTCAGTGGCCAAAACACAGGCAGGCAGACAGGCAGTC) |

| Negative control | sequence |
|---|---|
| Negative-F | tgctgAAATGTACTGCGCGTGGAGACGTTTTGGCCACTGACTGACGTCTCCACGCAGTACATTT ;A1 ; multiple cloning site ; C ; multiple cloning site ; |
| | (SEQ ID NO.: 6, tgctgAAATGTACTGCGCGTGGAGACGTTTTGGCCACTGACTGACGTCTCCACGCAGTACATTT) |
| Negative-R | cctgAAAATGTACTGCCGTGGAGACGTCAGTCAGTGGCCAAAACGTCTCCACGCCGCAGTACATTTc ; A2 ; multiple cloning site ; C ; multiple cloning site ; |
| | (SEQ ID NO.: 7, cctgAAATGTACTGCGTGGAGACGTCAGTCAGTGGCCAAAACGTCTCCACGCGCAGTACATTTc) |

### 2.2 The construction and validation of the miRNA vector

### 2.2.1 Materials

BLOCK-iT™ Pol II miR RNAi expression vector cassette containing EmGFP (purchased from Life)
Oligo DNA (purchased from Life)
Competent cell DH5α (purchased from Life)

### 2.2.2 Methods

### (1) Annealing

The synthesised 2 pairs of oligo single-stranded DNAs were dissolved in ddH₂O to 100µM, and 5µl of each of the complementary single strands were taken and mixed pairwise, and annealed in the system given in Table 2. 2 portions of the oligo mixture were heated at 95°C for 5 minutes, and then placed at room temperature for 20 minutes to form double-stranded DNAs.

**Table 2. Oligo DNA annealing system**

| | |
|---|---|
| 100µM top strand oligo | 5 µl |
| 100µM bottom strand oligo | 5 µl |
| 10×oligo annealing buffer | 2 µl |
| ddH₂O | 8 µl |
| Total volume | 20 µl |

### (2) Ligation

The annealed double-stranded DNAs were then diluted to a concentration of 10 nM, and ligated at room temperature in the system given in Table 3 for 30 minutes.

**Table 3. Enzyme ligation system**

| | |
|---|---|
| 5×ligation buffer | 4 µl |
| pcDNA6.2-GW/EmGFP-miR Or pcDNA6.2-GW/miR | 2 µl |
| ds oligo (10 nM) | 4 µl |
| T4 DNA ligase(1 U/µl) | 1 µl |
| ddH₂O | 9 µl |
| Total volume | 20 µl |

| | |
|---|---|
| Note: the structures of pcDNA6.2-GW/EmGFP-miR and pcDNA6.2-GW/miR are as shown in Figure 1. | |

### (3) Transformation

100 µl competent cells of DH5α were transformed with 10 µl of ligated product, followed by spreading on LB plates (containing 50 µg/ml spectinomycin) and incubating at 37°C.

### (4) Sequencing and validation

3 clones were respectively picked from each transformation plate, followed by shaking same and extracting plasmids therefrom, and sequencing to validate whether the inserted fragment sequence in the recombinant clones was consistent with the designed oligo single-stranded DNA sequence or not.

### Example 2: Cell experiment for validating the efficiency of the over-expression of anti-miR-214

A549 cells (purchased from Cell Resource Centre, Shanghai Institute for Biological Sciences of the Chinese Academy of Sciences) were transfected with the anti-miR-214 plasmids, the total RNAs were collected, and then the levels of anti-miR-214-5p, anti-miR-214-3p and miR-214 were detected by Real-time-PCR using primers for anti-miR-214-5p, anti-miR-214-3p and miR-214 respectively, customised from ABI Corporation. The results were presented as Ct values, which represent the cycle number undergone when the fluorescence signal in each reaction tube reached a set threshold, wherein there was a linear relationship between the Ct value of each template and the logarithm of the initial copy number of the template, i.e., the larger the initial copy number, the less the Ct value, and the less the initial copy number, the larger the Ct value. Table 4 shows the assay for the expression levels of anti-miRNA and miRNA using Real-time PCR.

**Table 4. The expression levels of anti-miR-214-5p, anti-miR-214-3p, and miR-214**

| | |
|---|---|
| (Ct) | anti-miR-214-5p |
| Blank plasmid | 39.63±0.2928 |
| anti-miR-214 plasmid | 24.55±0.5052 |
| | |
| (Ct) | anti-miR-214-3p |
| Blank plasmid | 30.45±0.7865 |
| anti-miR-214 plasmid | 33.87±0.5683 |
| | |
| (Ct) | miR-214 |
| Blank plasmid | 20.55±0.6822 |
| anti-miR-214 plasmid | 23.63±0.5119 |

Conclusion: after the A549 cells were transfected with the anti-miR-214 plasmids, a sharp increase in the anti-miR-214-5p can be detected (p<0.01), the anti-miR-214-3p cannot be substantially detected (p > 0.05), and a rise in miR-214 could not be caused, and on the contrary, a decrease in miR-214 was caused (p<0.05). Based on the difference of the Ct values, it can be determined that the ratio of F5 to F3 is much more than 50/1.

### Example 3: In vivo experiment for validating the efficiency of the over-expression of anti-miR-214

The C57/BL6 mice were administered with the anti-miR-214 plasmids by intravenous tail injection, and perfused after 24 h, followed by taking blood, heart, liver, spleen, lung, kidney, brain and muscle tissues, collecting the total RNAs, and then performing q-PCR for detecting the levels of the anti-miR-214-5p, anti-miR-214-3p and miR-214. The results can be seen in Table 5.

**Table 5. The expression levels of anti-miR-214-5p and anti-miR-214-3p**

| (Ct) | anti-miR-214-5p | anti-miR-214-3p |
|---|---|---|
| Blank mouse blood | 38.80±0.4171 | 30.80±0.8066 |
| 0.1 mg plasmids, in mouse blood | 24.11±0.4588 | 30.74±0.1667 |
| 0.01 mg plasmids, in mouse blood | 28.09±0.9880 | 30.50±0.1404 |
| Blank mouse liver | 39.37±0.8329 | 30.61±0.1288 |
| 0.1 mg plasmids, in mouse liver | 20.52±0.3156 | 30.67±0.1311 |
| 0.01 mg plasmids, in mouse liver | 25.30±0.1404 | 33.70±0.1351 |
| Blank mouse lung | 39.25±0.5662 | 33.12±0.1290 |
| 0.1 mg plasmids, in mouse lung | 26.37±0.3754 | 30.08±0.1283 |
| 0.01 mg plasmids, in mouse lung | 30.76±0.1895 | 33.58±0.1387 |

The experimental results suggested that:
it can be seen from the experimental results that after injecting with 0.1 mg plasmids, the levels of anti-miR-214-5p in mouse whole blood, liver, and lung have been significantly increased (p<0.05), and a change in anti-miR-214-3p cannot be detected (p< 0.05).

### Example 4: In vivo experiment for validating the metabolism kinetics of the over-expressed siRNAs

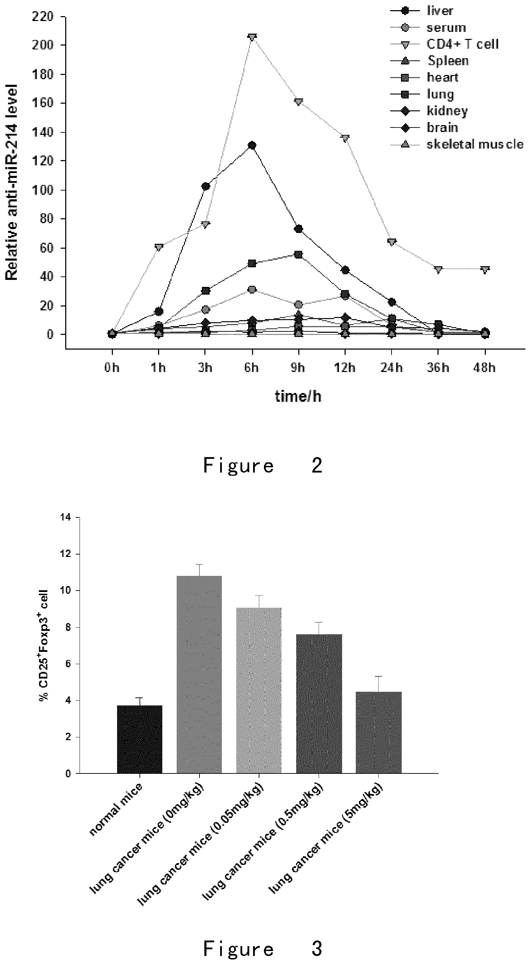

The mice were administered with the anti-miR-214 over-expression plasmid by intravenous tail injection (in the following experiments, the anti-miR-214 always refers to anti-miR-214-5p), the siRNA amounts in the plasma, heart, liver, spleen, lung, kidney, brain, skeletal muscle and CD4+T cells were detected after 1 h, 3 h, 6 h, 9 h, 12 h, 24 h, 36 h and 48 h, and the detailed results are shown in Figure 2. It can be seen from Figure 2 that siRNAs can actually enter the lung and reach a peak concentration after 6 h, and can be detected within 24 h.

### Example 5: Experiment in tumor-bearing mice

A lung cancer metastasis model was constructed by injecting LLC cells from the tail-vein of mice, and after the model was successfully constructed, the anti-miR-214 over-expression plasmid was administered by intravenous tail injection, and the therapeutic effect was observed. After the mice were sacrificed, various tissues of the mice were taken for detecting the distribution of anti-miR-214 in various tissues by Real-time PCR, and tissue sections were made for detecting and observing the therapeutic effect.

### (1) The change in the quantity of CD25⁺ and Foxp3⁺ T cells

The detailed results are shown in Figure 3. It can be seen from Figure 3 that the anti-miR-214 plasmid can effectively inhibit the quantity of new T cells CD25⁺ and Foxp3⁺ T cells (Treg) in the tumor-bearing mice in a dose-dependent manner, and as the concentration of the injected plasmids increases, the quantity of CD25⁺ and Foxp3⁺ T cells (Treg) decreases.

### (2) The amounts of anti-miR-214 in various tissues

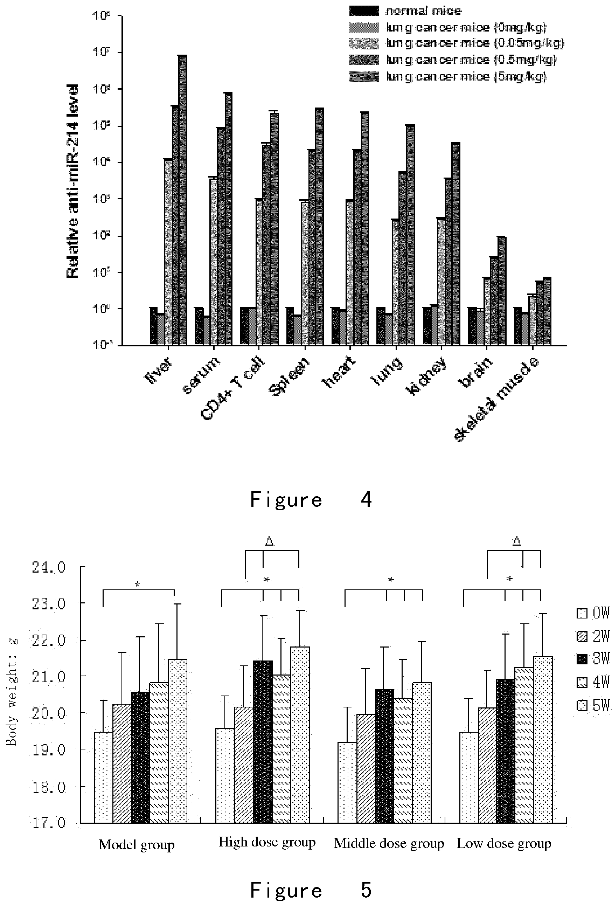

The detailed results are as shown in Figure 4. It can be seen from Figure 4 that after injecting with the anti-miR-214 plasmid, anti-miR-214 can be delivered by the liver to various tissues, resulting in a sharp increase in the level of anti-miR-214 in various tissues.

### Example 6: The study of the therapeutic effect of anti-miR-214 on the mouse Lewis lung cancer

By constructing an *in vivo* tumor model in C57BL/6 mice for studying the therapeutic effect of anti-miR-214 administered intravenously on the mouse Lewis lung cancer, reference can be provided for the later studies.

The LCC tumor cells were collected at the logarithmic phase, and taken for intravenous tail injection at 1×10⁶/mouse to construct an *in situ* Lewis lung cancer model. After the model was successfully constructed, a series of concentrations of anti-miR-214 were administered by intravenous injection, and the animal living conditions were observed during the administration period. After the administration was finished, the lung was taken for pathological section examination, and the severity of lung tumors and the therapeutic effect of anti-miR-214 were observed.

### 1. Experimental materials and methods

### 1.1 Experimental materials

Test compound: Anti-miR-214, content: 6.4 mg/ml, provided by School of Life Sciences, Nanjing University. The compound was diluted to a desired concentration with normal saline for injection in experiments.

LCC cell line: provided by School of Life Sciences, Nanjing University. DMEM is a product from Hyclone Corporation. Foetal calf serum is a product from Gibco Corporation. In the experiments, the LCC cell line was cultured in a DMEM complete media containing 10% FBS, 100 ug/ml penicillin and 100 ug/ml streptomycin, in an incubator at 37°C and 5% CO₂.

Animals: 40 C57BL/6 mice, half male and half female, 6-weeks old, provided by the Experimental Animal Centre, Yangzhou University, with a certification number of SCXK (Su) 2007-0001.

### 1.2 Experimental methods

LCC cells grown to the logarithmic phase were digested with pancreatin, followed by centrifuging at 1000 rpm, discarding the supernatant, washing twice with sterile normal saline, suspending the cells in normal saline, trypan blue staining for observing the cell viability, performing cell counting, and adjusting the cell density at 5×10⁶/ml. In the experiments, healthy C57BL/6 mice were taken, and injected with 0.2 ml/mouse slowly through the tail-vein, and after the injection was finished, all the modelled mice were classified into a model group (negative control group), high dose group (5 mg/kg), middle dose group (0.5 mg/kg), and low dose group (0.05 mg/kg). During the construction of the model, the spirit, dietary status, defecation, body weight, activity and other conditions of the C57BL/6 mice were observed periodically. Starting from day 14, the mice were administered with 0.1 ml/10 g body weight by intravenous tail injection, and the control group was administered with the corresponding amount of normal saline. During administration, the mice were administered with same once every 3 days, and 7 times in total. At day 3, after the last administration, the mice were anaesthetised with diethyl ether, followed by taking the blood, lung and liver. The lung and liver were placed in 10% formalin, pathological sections were made, and the lung cancer model construction situation and the treatment situation of anti-miR-214 on the lung cancer were observed.

### Lung tumor pathological sections and scoring

Pathological sections for all the lung tissues were observed. According to the general situation observed by the naked eye, sections of 3 different lesion sites were made, and according to the lung tumor formation situations and severity, the sections were classified into "-" meaning that tumor lesions were not seen under microscope; "±" meaning slight tumor lesions; "+" meaning mild tumor lesions; "++" meaning moderate tumor lesions; "+++" meaning severe tumor lesions, and scored as 0-4 respectively.

### 1.3 Statistical processing

All the measurement data were expressed as χ̅±SD. An SPSS 16.0 software package was used for statistical analysis and processing, comparison among multiple groups was performed with a variance analysis F test, and comparison among groups was performed with a grouping t test, with P<0.05 as having statistical significance.

### 2. Results

### 2.1 Observation of general situations of animals during model construction and medicament administration

During model construction, the living conditions of all the animals were good, and adverse effects such as piloerection, a dull look in the eyes, abnormal respiration, slow activity and abnormal stools were not seen. Two weeks after the model construction, the animals' body weights were all increased, but without statistical difference as compared with those before the model construction (P>0.05). During administration, abnormal responses caused by the medicament were not seen in all animals in each administration group. During administration, all the animal's body weights showed an increase to different extents, and the body weight gain in the administration groups was obvious. As compared with the body weights before the model construction, there is a significant statistical difference starting from week 3 (P<0.05); and the increase in the negative control group (i.e. model group) was relatively slow, and shows the difference to week 5 from the body weights before the administration. The body weights of animals in the high and low dose groups had significant statistical differences (P<0.05) as compared with the body weights before the administration (i.e. week 2). The detailed results are shown in Figure 5.

### 2.2 The therapeutic effect of anti-miR-214 on the mouse Lewis lung cancer

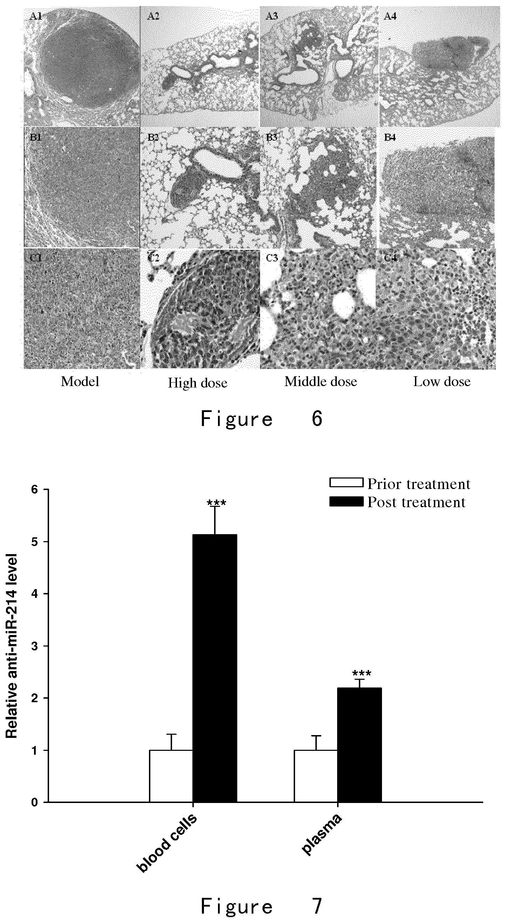

2 weeks after the C57BL/6 mice were used for Lewis lung cancer model construction, anti-miR-214 was administered by intravenous injection for treatment, and during administration, the mice were administered with same once every 3 days, and the animals were sacrificed at day 3 after the final administration, for taking the blood, lung and liver. A part of the lung was used for measurement of the amount of anti-miR-214, and the remaining lung and the liver were fixed with formalin, and pathological tissue sections were made for examining the tumor situations of the organs. The detailed results are shown in Figure 6. It can be seen from the results of Figure 6 that the pathological sections show that tumor lesions were not seen in all the liver sections in each group. In the lung, tumor cell foci with a flake-shaped nucleus being stained largely and deeply to different extents can be seen in each treatment group. In each of the administration groups, a high dose, i.e. 5 mg/kg, of micro-214 had a good therapeutic effect on the *in vivo* Lewis lung cancer, with the score and severity being less than those of the control group, and the number of mouse individuals where tumor lesions were not seen under a light microscope was also more than that of the control group; and the slow dose group, i.e. 0.05 mg/kg, also had a certain therapeutic effect on the *in situ* Lewis lung cancer, which was possibly related with the *in vivo* replication of the medicament, and the results can be seen in Tables 6 and 7.

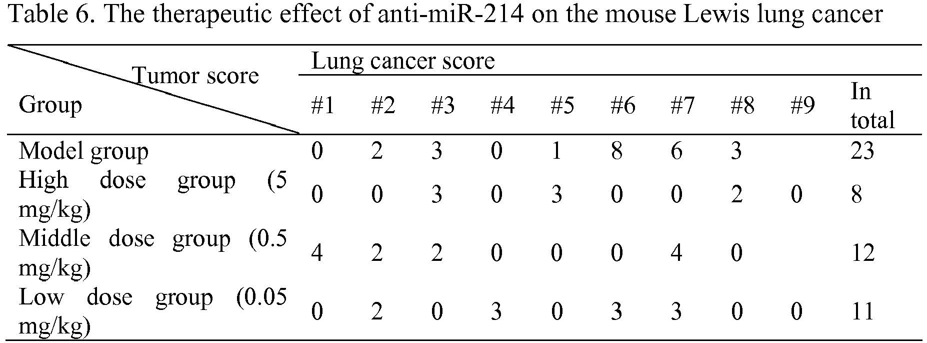

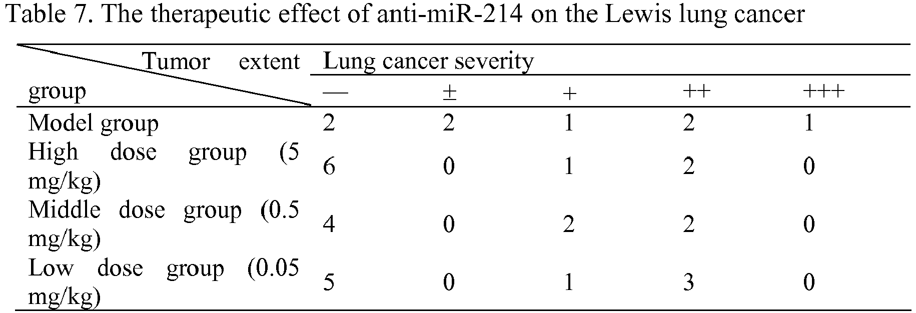

### 3. Conclusions

During administration, the living conditions of all the animals were good, abnormal responses related with the medication were not seen, and the body weights of the animals in the administration groups were obviously increased and showed a significant difference as compared with the body weights before the model construction or administration (P<0.05). Pathological sections showed that the tumor severity in each administration group was reduced further than that in the control group, and the tumor inhibition effect of anti-miR-214 was obvious at 5 mg/kg.

Anti-miR-214 had a certain dose-dependent therapeutic effect on the mouse Lewis lung cancer *in vivo,* wherein the therapeutic effect was the most obvious at a concentration of 5 mg/kg, and the abnormal responses related with the medication were not seen during administration.

### Example 7. Clinical trials

Volunteers with end-stage breast cancer metastasis were injected with the anti-miR-214 plasmid, with an infusion twice per week of 1 mg each time. Each indicator was detected in the volunteers after medication for one month.

### (1) Detection of anti-miR-214

The detailed results are shown in Figure 7. It can be seen from Figure 7 that after the volunteers with end-stage tumors were injected with the plasmid (after treatment), high contents of anti-miR-214 can be detected in both the blood cells and the plasma in the volunteers with end-stage tumors, which sufficiently suggested that the intravenous injection of the anti-miR-214 plasmid can actually result in high expression of anti-miR-214 *in vivo.*

### (2) Results of miR-214 in the serum

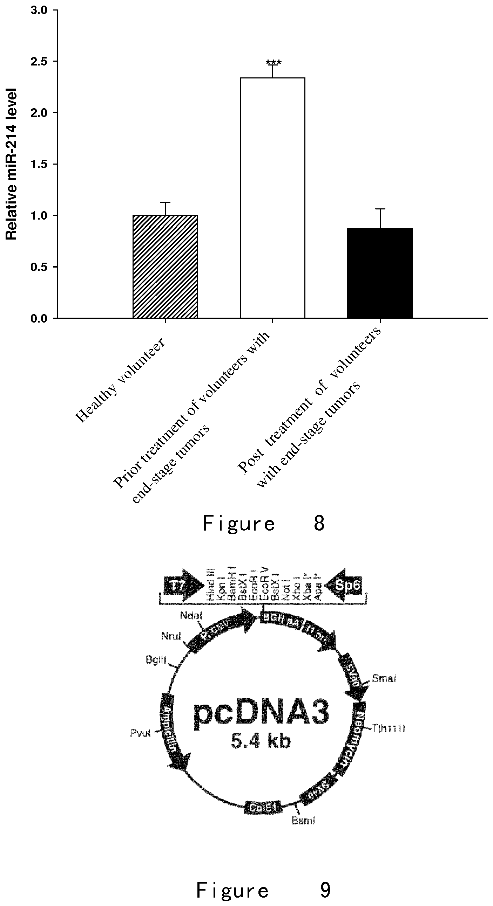

Figure 8 shows the expression level of miR-214 in the serum. It can be seen from Figure 8 that after injecting with the plasmid, the miR-214 in the sera of the volunteer patients was significantly decreased compared with that before the treatment, and almost decreased to a comparable level as that in a normal human, which suggests that the anti-miR-214 plasmid injected was not only expressed *in vivo,* but also functioned to adsorb the miR-214 *in vivo* so as to significantly decrease its expression level.

### (3) Changing situations of tumors

After injecting with the anti-miR-214 plasmid, the conditions of the volunteers with end-stage breast cancer metastasis were improved to some extent, but the tumor size and volume were not changed, and tumor metastasis did not occur; it can be seen therefrom that the miR-214 inhibitor had an inhibitory effect on tumor growth, and the miR-214 inhibitor can be used in tumor treatment.

### Example 8 Precursor miRNAs adapted for other over-expression vectors

The expression module constructed by inserting the precursor miRNA structure into the expression vector pcDNA3 likewise effectively avoids the problem that -3p and -5p are produced when some endogenous miRNAs are expressed. The profile structure of pcDNA3 was as shown in Figure 9.

### 1. Anti-miR-214-5p sequence

> anti-miR-214-5p
5'-ACUGCCUGUCUGUGCCUGCCUGU-3' (SEQ ID NO.: 2)

### 2. Anti-miR-214 vector

### 2.1. Design and synthesis of Oligo DNAs

According to anti-miR-214, 2 pairs of complementary oligo DNAs, i.e. the structures of the precursor miRNAs, were designed and synthesised as described in point 2.1 of Example 1.

### 2.2 The construction and validation of the miRNA vector

The particular operating procedures are as described in point 2.2 of Example 1. The method for validating the efficiency of the over-expression of anti-miR-214 was the same as that in Example 2. Table 8 shows the assay for the expression levels of anti-miRNA and miRNA using Real-time PCR.

**Table 8. The expression levels of anti-miR-214-5p, anti-miR-214-3p, and miR-214**

| | |
|---|---|
| (Ct) | anti-miR-214-5p |
| Blank plasmid | 38.25±0.2564 |
| anti-miR-214 plasmid | 25.43±0.5123 |
| | |
| (Ct) | anti-miR-214-3p |
| Blank plasmid | 31.25±0.4585 |
| anti-miR-214 plasmid | 32.89±0.6245 |
| | |
| (Ct) | miR-214 |
| Blank plasmid | 21.03±0.5874 |
| anti-miR-214 plasmid | 24.08±0.1254 |

Conclusion: after the A549 cells were transfected with other over-expression vector plasmids constructed using the precursor miRNAs, a sharp increase in the anti-miR-214-5p can be detected (p<0.01), the anti-miR-214-3p cannot be detected, and a rise in miR-214 could not be caused, and on the contrary, a decrease in miR-214 was caused (p<0.05). Inserting the precursor miRNA structure into other expression vectors likewise effectively avoids the problem that -3p and -5p are produced when some endogenous miRNAs are expressed.

### Comparative example 1

The precursor miRNAs which are different from those in the present invention were constructed using anti-miR-214, and the constructed precursor miRNAs were inserted into the vector pcDNA™ 6.2 identical to that in the present invention. A549 cells were transfected with the anti-miR-214 plasmids, the total RNAs were collected, and then the levels of anti-miR-214-5p, anti-miR-214-3p and miR-214 were detected by q-PCR using primers for anti-miR-214-5p, anti-miR-214-3p and miR-214 respectively, customised from ABI Corporation.

### 1. Anti-miR-214-5p sequence

> anti-miR-214-5p
   5'-ACUGCCUGUCUGUGCCUGCCUGU-3' (SEQ ID NO.: 2)

### 2. Anti-miR-214 vector

### 2.1. Design and synthesis of Oligo DNAs

According to the gene sequences, 2 pairs of complementary oligo DNAs were designed and synthesised, and for the sequences, reference can be made to Table 9.

The oligo structures designed and synthesised are as follows:

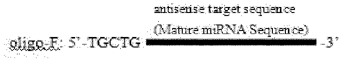
antisense target sequence
(Mature miRNA Sequence)
oligo-F: 5'-TGCTG -3'

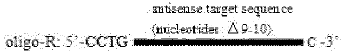
antisense target sequence
(nucleotides Δ9-10)
oligo-R: 5'-CCTG C-3'

**Table 9. The oligo DNA sequences and their corresponding precursor miRNA elements**

| Oligo name | Oligo DNA sequence 5'-3' |
|---|---|
| Mature sequence of >anti-miR-214-5p: 5'-ACUGCCUGUCUGUGCCUGCCUGU-3' (SEQ ID NO.: 2) | |
| 13MR0041-1F | TGCTGGTTTTGGCCACTGACTGAC (SEQ ID NO.: 8) |
| 13MR0041-1R | CCTGGTCAGTCAGTGGCCAAAACC (SEQ ID NO.: 9) |

| Negative control sequence | |
|---|---|
| Negative-F | (SEQ ID NO.: 6, tgctgAAATGTACTGCGCGTGGAGACGTTTTGGCCACTGACTGACGTCTCCACGCAGTACATTT) |
| Negative-R | (SEQ ID NO.: 7, cctgAAATGTACTGCGTGGAGACGTCAGTCAGTGGCCAAAACGTCTCCACGCGCAGTACATTTc) |

### 2.2 The construction and validation of the miRNA vector

The particular operating procedures are as described in point 2.2 of Example 1. The results can be seen in Table 10.

**Table 10. The expression levels of anti-miR-214-5p, anti-miR-214-3p, and miR-214**

| | |
|---|---|
| (Ct) | anti-miR-214-5p |
| Blank plasmid | 35.92±0.1545 |
| anti-miR-214 plasmid | 26.45±0.1535 |
| | |
| (Ct) | anti-miR-214-3p |
| Blank plasmid | 32.12±0.1657 |
| anti-miR-214 plasmid | 25.48±0.1456 |
| | |
| (Ct) | miR-214 |
| Blank plasmid | 21.26±0.5652 |
| anti-miR-214 plasmid | 21.43±0.4586 |

It can be seen from Table 10 that after A549 cells were transfected with precursor miRNA plasmids which are different from those in the present invention, a sharp increase in anti-mlR-214-5p can be detected (p<0.05); however, the expression level of anti-miR-214-3p was also very high (p<0.05), and the level of miR-214 was not significantly changed. This is because the precursor miRNA plasmid which is different to that in the present invention can not only express anti-miR-214-5p, but can also express anti-miR-214-3p, and due to competitive binding, the mass anti-miR-214-5p was adsorbed by anti-miR-214-3p, so that the expression level of miR-214 cannot be decreased.

### Example 9

Examples 1 and 2 were repeated, except for that the structure of A2 was changed from CAGG to CAGGA.

The results indicated that the change in the structure of A2 from CAGG to CAGGA resulted in a further increase of the ratio of F5 to F3 by about 50%. This suggested that the use of CAGGA were more helpful for improving the ratio of F5 to F3.

All the documents mentioned in the present invention are referred to by incorporation, as well as alone. In addition, it should be understood that after reading the teachings of the present invention as described above, a skilled person in the art can make various changes or modifications to the invention, and these equivalent forms shall also fall into the scope of the present application as defined by the appended claims.

## Claims

1. A pharmaceutical preparation, **characterised in that** the preparation comprises:
(a) an expression vector for expressing anti-miRNAs and/or siRNAs; and
(b) a pharmaceutically acceptable carrier.

2. A method for administering a medicament, **characterised in that** the method comprises the step of:
administering the pharmaceutical preparation of claim 1 at a first site on a mammal, so that the expression vector is processed to form a microvesicle in the mammal which is transported to a second site on the mammal where the anti-miRNAs and/or siRNAs are expressed.

3. A precursor sequence, **characterised in that** the sequence has a structure from the 5' terminus to the 3' terminus as shown in formula I: 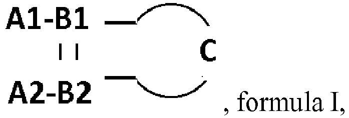
wherein B1 is a first ribonucleic acid sequence as desired, and comprises an anti-miRNA sequence form or an siRNA sequence form;
B2 is a sequence substantially or completely complementary to B1, and B2 is not complementary to C;
C is a stem-loop structure sequence; and
A1 and A2 are null or are optionally RNA sequences consisting of 4-5 bases respectively;
wherein the precursor can express (or produce or enrich) the first ribonucleic acid sequence in a host, but cannot express (or produce or enrich) a second ribonucleic acid sequence complementary to the first ribonucleic acid sequence.

4. The precursor sequence of claim 3, **characterised in that** the sequence has a structure from the 5' terminus to the 3' terminus as shown in formula I: 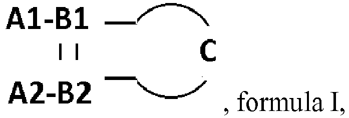
wherein B1 is anti-miRNA-214-5p;
B2 is a sequence substantially or completely complementary to B1, and B2 is not complementary to C;
C is a stem-loop structure sequence, preferably a sequence shown as SEQ ID NO.: 1; and
A1 and A2 are null or are optionally RNA sequences consisting of 4-5 bases respectively;
wherein the precursor sequence as shown can be processed in the host to form anti-miRNA-214, and only the anti-miRNA-214-5p, rather than the anti-miRNA-214-3p, in the anti-miRNA-214 is expressed.

5. The precursor sequence of claim 4, **characterised in that** substantially complementary means that there are 2-8 non-complementary bases between the B2 and B1, preferably there are 3-5 non-complementary bases between the B2 and B1, and more preferably 1-2 bases are deleted in B2 as compared with B1.

6. The precursor sequence of claim 4, **characterised in that** A1 is UGCUG; and/or A2 is CAGG or CAGGA.

7. A polynucleotide, **characterised in that** the polynucleotide can be transcribed by a host to form the precursor sequence of claim 4.

8. An expression vector, **characterised in that** the expression vector contains the precursor sequence of claim 4 or the polynucleotide of claim 7.

9. A pharmaceutical composition, **characterised in that** the pharmaceutical composition comprises the precursor sequence of claim 4 or the expression vector of claim 8, and a pharmaceutically acceptable carrier.

10. The pharmaceutical composition of claim 9, **characterised in that** the pharmaceutical composition is the expression vector of claim 8, and preferably is a plasmid containing the precursor sequence of claim 4; and/or
the dosage form of the pharmaceutical composition comprises a tablet, a capsule, a powder, a pill, a granule, a syrup, a solution, a suspension liquid, an emulsion, a suspension, an injection solution, or an injectable powder; preferably the dosage form is an injection, such as an intravenous injection or an intraperitoneal injection.

11. The pharmaceutical composition of claim 9, **characterised in that** the administration mode of the pharmaceutical composition comprises oral, respiratory tract, injection, transdermal, mucosal, or cavity administration; preferably the administration mode comprises direct injection of a plasmid.

12. A use of the precursor sequence of claim 4 or the expression vector of claim 8, **characterised in that** the use is: (i) for preparing an inhibitor of miRNA-214; and/or (ii) for preparing a pharmaceutical composition against a malignant tumor highly expressing miRNA-214;
preferably, the malignant tumor comprises liver cancer, lung cancer, stomach cancer, oesophageal cancer, ovarian cancer, colorectal cancer, cervical cancer, pancreatic cancer, prostatic cancer, leukaemia or breast cancer.
